# EUROPEAN PATENT SPECIFICATION

(11) **EP 3 399 034 B1**
(45) Date of publication and mention of the grant of the patent: **28.12.2022**
(21) Application number: 17169666.9
(22) Date of filing: 05.05.2017
(51) Int. Cl.: C12N 15/10

(54) **DEVICE FOR EXTRACTING NUCLEIC ACIDS FROM BIOLOGICAL SAMPLE MATERIALS WITH SOLVENT-FREE REAGENTS**
VORRICHTUNG ZUR EXTRAKTION VON NUKLEINSÄUREN AUS BIOLOGISCHEN PROBENMATERIALIEN MIT LÖSUNGSMITTELFREIEN REAGENZIEN
DISPOSITIF PERMETTANT D'EXTRAIRE DES ACIDES NUCLÉIQUES À PARTIR DE MATÉRIAUX D'ÉCHANTILLON BIOLOGIQUE À L'AIDE DE RÉACTIFS EXEMPTS DE SOLVANT

(43) Date of publication of application: 07.11.2018
(73) Proprietor: Siemens Healthcare Diagnostics Inc., Tarrytown, NY 10591-5098 (US)
(72) Inventor: Gumbrecht, Walter, 91074 Herzogenaurach (DE); Huang, Yiwei, 91058 Erlangen (DE); Paulicka, Peter, 91341 Röttenbach (DE)
(74) Representative: Plate, Jürgen

(56) References cited:
- WO-A1-2005/106024
- WO-A1-2006/085104
- WO-A1-2012/028880
- WO-A1-2015/120445
- WO-A2-2005/089929
- WO-A2-2006/023471
- WO-A2-2006/071770
- CN-A- 101 297 037
- CN-A- 105 695 624
- US-A1- 2006 078 923
- US-A1- 2007 059 751
- US-B2- 7 527 929
- MARIA MORENO-GUZMAN ET AL: "Disposable immunosensor for cortisol using functionalized magnetic particles", ANALYST, vol. 135, no. 8, 1 January 2010 (2010-01-01), page 1926, XP055382978, ISSN: 0003-2654, DOI: 10.1039/c0an00206b
- MAGORZATA A WITEK ET AL: "96-Well Polycarbonate-Based Microfluidic Titer Plate for High-Throughput Purification of DNA and RNA", ANALYTICAL CHEMISTRY, AMERICAN CHEMICAL SOCIETY, US, vol. 80, no. 9, 1 May 2008 (2008-05-01), pages 3483-3491, XP002693159, ISSN: 0003-2700, DOI: 10.1021/AC8002352 [retrieved on 2008-03-21]

## Description

The present invention relates to a device comprising a container having an inner wall and at least one opening, containing a mixture containing at least one reagent, and at least one additive, a method of producing this device, and the use of the device for enriching and/or nucleic acids in a sample.

In molecular diagnostics sample preparation is of particular importance since - in contrast to clinical-chemical (CC) or immunoassays (IA) - the sample material, e.g. blood plasma, cannot be examined directly, but a material to be examined, e.g. a nucleic acid, has to be first extracted, e.g. from human cells, bacteria or viruses, and cleaned.

WO 2012/028880 A1 discloses a method and an apparatus for extracting a nucleic acid analyte from a biological sample.

WO 2005/106024 A1 relates to a method and device for isolating nucleic acid using a cartridge that contains dry and partly water-soluble reagents.

WO 2015/120445 A1 describes a process, a composition and a test kit for isolation of cell free nucleic acid and analogs thereof wherein the composition comprises a solid chaotropic salt that is used in conjunction with commercially available magnetic beads having substrates made from functionalized polyvinyl alcohol, polystyrene and SiO₂.

WO 2006/023471 A2 is directed to a system and a method employing a chamber with a therein disposed additive which comprises at least one chaotropic substance, at least one detergent, and at least one buffer.

Moreno-Guzman et al. (Disposable immunosensor for cortisol using functionalized magnetic particles; Analyst (2010) 135: 1926-1933) describe an electrochemical immunosensor for cortisol using screen-printed carbon electrodes and functionalized magnetic beads.

US 2007/059751 A1 pertains to a method and device for isolating nucleic acid from biological samples wherein the sample is disrupted with a mechanism in order to release and subsequently bind nucleic acid onto a solid phase carrier.

WO 2006/071770 A2 relates to a disposable device for nucleic acid analysis comprising magnetic beads with coating layers made from cross-linked polystyrene and a hydrophilic glycidyl ether with carboxylic acid groups, a separation barrier made from a wax through which the magnetic beads pass upon application of a magnetic field and a conduit containing a sensing region with an immobilized capture oligonucleotide.

WO 2005/089929 A2 relates to a method of separating polynucleotides, such as DNA, RNA and PNA, from solutions containing polynucleotides by reversibly binding the polynucleotides to a solid surface comprising a polymer selected from polyvinylpyrrolidone, polyethylene glycol and polyols other than cellulose.

CN 101 297 037 A discloses a method for inactivating an RNA degrading enzyme present in a sample and facile and reliable extraction and separation of RNA from cells, fungi, bacteria, viruses etc. present in the sample. The method further relates to an RNA amplification method using reverse transcription-polymerase chain reaction (RT-PCR).

WO 2006/085104 A1 pertains to a method and kit for isolating nucleic acid from a sample, wherein the sample is contacted with magnetic beads in the presence of an ethylene glycol multimer consisting of from 2 to 70 ethylene oxide monomers, the soluble nucleic acid is bound to the magnetic beads, and the magnetic beads and the thereto bound nucleic acids are separated from the sample.

M. A. Witek, M. L. Hupert, D. S.-W. Park, K. Fears, M. C. Murphy, S. A. Soper; 96-Well Polycarbonate-Based Microfluidic Titer Plate for High-Throughput Purification of DNA and RNA; Anal Chem. 2008 May 1; 80(9): 3483-3491. doi:10.1021/ac8002352 describe a method for high-throughput purification of nucleic acids from whole cell lysates or whole blood using a photactivated polycarbonate solid-phase reversible immobilization microfluidic chip.

A number of established techniques exist for this purpose, which are based on the usage of e.g. detergents (membrane lysis), chaotropic salts (protein denaturation), proteases (enzymatic digestion), and magnetic beads, e.g. silica beads (bonding of nucleic acids). In all cases the reagents are present in liquid or dissolved/suspended form (mostly aqueous). This involves extensive pipetting steps in these methods, respectively workflows, for nucleic acid extraction being necessary, either manually or automated. This leads to a high usage of pipette tips (cost, waste) and an enormous amount of time spent. Also logistical requirements, like cool storage, expiry dates, and treatment of opened packages or containers, have to be considered.

A further problem is based on requirements regarding the usage of reagents. For example, chaotropic agents, e.g. salts like guanidinium thiocyanate, have to be used in very high final concentrations in presence of a sample liquid, but the solubility thereof in the reagent solution is limited. As a consequence a high reagent volume (Vᵣ) is necessary for treating a certain sample volume (Vₚ), which can surpass it by a manifold (Vᵣ>Vₚ). A consequence thereof is a reduced sample volume and therefore a reduced yield.

A partial simplification of the work flow and logistics can be achieved for some producers by providing reagents necessary for processing a single sample in liquid form in individual compartments of a plastic cassette, e.g. reagent cassettes with liquid reagents for nucleic acid extraction, e.g. by Roche MagNA Pure and Becton Dickinson BD MAX^{™}. For removing the reagent, seal membranes thereof can be e.g. pierced by a pipette tip. However, this approach does not solve the problems of logistics and limiting of sample volume.

Therefore there is a need for improved methods and devices for extracting a substance from a sample and/or enriching in a sample and/or detecting a substance in a sample, wherein particularly the amount of pipetting and/or solvents is reduced.

The inventors found a device for extracting a substance from a sample and/or enriching in a sample and/or detecting a substance in a sample in which the use of pipetting steps and solvent use can be minimized or - in case of solvent use - even avoided by fixing the reagents in a container inside a matrix of an additive.

With the present device the need for a solvent like water in the above procedures can be reduced or even eliminated, e.g. when using reagents, particularly when the sample itself contains a liquid like water. For example, in blood sample testing blood plasma consists of about 90 wt.% of water.

Furthermore, also the storage stability of the reagents can be improved due to avoiding solvents. In addition, no measures are necessary to avoid evaporation of solvents, making the procedures easier and more reliable. Also for analytical purposes this is of particular importance. Apart from that, also logistics can be simplified as a sample can be used as is and pipetting steps can be reduced. Particularly, pipetting of reagent solutions can be reduced or even totally avoided, reducing the amount of time, tips, and waste in the procedures. Apart from that, bigger sample amounts are possible to use with constant reagent concentration.

In a first aspect the present invention relates to a device comprising a container (1) having an inner wall (2) and at least one opening, containing a mixture (3) containing
- at least one reagent comprising beads (4), and
- at least one additive (5) that is in solid form at room temperature,
   wherein the at least one reagent is fixed to at least part of the inner wall (2) of the container (1) with the at least one additive (5),
   the at least one reagent is dispersed in the at least one additive (5), and
   the at least one additive (5) is a water-soluble polymer selected from polyethylene glycol, polypropylene glycol, polyvinylpyrrolidone, polyvinylalcohol and/or copolymers thereof.

Furthermore disclosed herein is a method of extracting a substance from a sample, enriching a substance in a sample and/or detecting a substance in a sample, comprising
- providing the present device;
- introducing the sample into the container; and
- mixing the sample with the mixture containing the at least one reagent, and the at least one additive. This method of extracting a substance from a sample is not part of the invention.

In addition, a further aspect relates to a method of producing the present device, comprising:
- providing a container (1) having an inner wall (2) and at least one opening;
- preparing a mixture (3) containing at least one reagent comprising beads (4) and at least one additive (5) that is in solid form at room temperature; and
- introducing the mixture (3) in the container (1), wherein the at least one reagent is fixed to at least part of the inner wall (2) of the container (1) with the at least one additive,

the at least one reagent is dispersed in the at least one additive (5), and
the at least one additive (5) is a water-soluble polymer selected from polyethylene glycol, polypropylene glycol, polyvinylpyrrolidone, polyvinylalcohol and/or copolymers thereof.

Further aspects and embodiments of the invention are disclosed in the dependent claims and can be taken from the following description, figures and examples.

### Figures

The enclosed drawings should illustrate embodiments of the present invention and convey a further understanding thereof. In connection with the description they serve as explanation of concepts and principles of the invention. Other embodiments and many of the stated advantages can be derived in relation to the drawings. The elements of the drawings are not necessarily to scale towards each other. Identical, functionally equivalent and acting equal features and components are denoted in the figures of the drawings with the same reference numbers, unless noted otherwise.
Fig. 1 shows a schematic drawing of an embodiment of the present invention.
Fig. 2 shows schematically the requirements of a method of the prior art.
Fig. 3 shows an Example of the present device.

### Detailed description of the invention

### Definitions

Unless defined otherwise, technical and scientific terms used herein have the same meaning as commonly understood by one of ordinary skill in the art to which this invention belongs.

In the context of the present invention a "sample" is a sample to be analyzed for a substance and/or which comprises a substance to be extracted and/or enriched, e.g. at least an analyte and/or a nucleic acid. According to certain embodiments the substance in the present method of extracting a substance from a sample, enriching a substance in a sample and/or detecting a substance in a sample is a nucleic acid and/or an analyte, e.g. a nucleic acid, particularly when the sample is extracted and/or enriched, or an analyte when the sample is analyzed.

The sample is not particularly restricted and can be of natural or synthetic origin and can e.g. comprise samples from simple or complex matrices, e.g. from a subject, e.g. a vertebrate, e.g. a human or animal, or from a natural source, e.g. an extract from a soil, air, or a natural water/water body. The sample can also be water-free or water-poor.

According to certain embodiments the sample is an aqueous sample, i.e. a sample containing water. For example, the sample can be a body fluid of a subject. Body fluids are thereby liquids originating from inside the bodies of subjects, particularly living subjects. They include fluids that can be excreted or secreted from the body and/or that circulate in the body, and body water. They can be in the state of a liquid, emulsion or suspension. Examples of body fluids within the invention are blood, urine, saliva, sputum, plasma, serum, etc. According to certain embodiments, the sample is a patient sample (clinical isolate). Exemplified samples are serum, plasma, and/or whole blood of a patient.

According to certain embodiments, the subject in the present method of extracting a substance from a sample, enriching a substance in a sample and/or detecting a substance in a sample can be a vertebrate, preferably a human or animal, more preferably a mammal and most preferred a human, respectively human patient.

A vertebrate within the present invention refers to animals having a vertebrate, which includes mammals - including humans, birds, reptiles, amphibians and fishes. The present invention thus is not only suitable for humans and the human medical field, but also for veterinary medicine.

The term "nucleic acid" refers to a polynucleotide molecule having a defined sequence. It comprises DNA molecules, RNA molecules, nucleotide analog molecules and combinations and derivatives thereof, such as DNA molecules or RNA molecules with incorporated nucleotide analogs or cDNA. It also comprises cell free (cf) DNA and RNA. The term "nucleic acid sequence" relates to the sequence of nucleotides in the nucleic acid molecule.

It must be noted that, as used in the specification and the appended claims, the singular forms "a," "an" and "the" include singular and/or plural referents unless the context clearly dictates otherwise. For example, the term "a" as used herein can be understood as one single entity or in the meaning of "one or more" entities. It is also to be understood that plural forms include singular and/or plural referents unless the context clearly dictates otherwise. It is moreover to be understood that, in case parameter ranges are given which are delimited by numeric values, the ranges are deemed to include these limitation values.

A first aspect of the present invention relates to a device comprising a container (1) having an inner wall (2) and at least one opening, containing a mixture (3) containing
- at least one reagent comprising beads (4), and
- at least one additive (5) that is in solid form at room temperature,

wherein the at least one reagent is fixed to at least part of the inner wall (2) of the container (1) with the at least one additive (5),
the at least one reagent is dispersed in the at least one additive (5), and
the at least one additive (5) is a water-soluble polymer selected from polyethylene glycol, polypropylene glycol, polyvinylpyrrolidone, polyvinylalcohol and/or copolymers thereof. With regard to the present invention, the inner wall of the container is a wall that can come into contact with the mixture containing at least one reagent and at least one additive, as well as with a sample that is introduced into the container. It can comprise e.g. the bottom in the inside of the container as well as inner side walls of the container. This means that the mixture containing at least one reagent and at least one additive can be fixed at the bottom and/or at the side walls in the inside of the container.

The container is not particularly restricted and can be in any form as long as it provides an inner wall and at least one opening, preferably one opening. It can be out of any suitable material and can take up any volume suitable for introducing a sample therein. It can be e.g. a tube, a well plate, a bottle, a beaker, a flask, etc., and can be in any shape. Preferably, the container has a volume that can be introduced of less than 500 mL, further preferably less than 200 mL, even further preferably less than 100 mL, e.g. 50, 20, 10, 5 or 2 mL or less.

According to certain embodiments the container is a tube, e.g. a test tube or a microtube, particularly a microtube, a cuvette, a well in a well plate, e.g. a microtiter plate, e.g. a 96-well titer plate; or a bottle. The device thus can be, according to certain embodiments, a well plate, e.g. a microtiter plate, e.g. a 96-well titer plate. According to certain embodiments, the container is not a dish, e.g. a Petri dish. Particularly tubes and/or cuvettes are advantageous as containers for use in e.g. high-throughput analyzation, extraction and/or enrichment, as a multitude of tubes and/or cuvettes, e.g. without a cap, can easily be filled, e.g. as bulk, respectively in loose form, into a high-throughput machine, e.g. a high-throughput analyzer, where they can be sorted and put upright before filling the device with a sample to be extracted, enriched and/or analyzed.

The container can be made of any material, e.g. ceramic, glass, metal, plastics, etc., as long as the material is suitable to take up the mixture containing at least one reagent and at least one additive, and preferably also suitable to have a sample to be analyzed, extracted and/or enriched introduced therein. Also, different parts of the container can be made of different material, e.g. an inner and an outer wall, or be made of the same material. It is also not excluded that the container or parts thereof are coated by a material before introducing the mixture containing at least one reagent and at least one additive. However, the container, or parts thereof, can also be uncoated. If a coating is provided the material for the coating is not particularly limited, and can e.g. comprise a substance for binding and/or fixing an analyte in a sample to be analyzed and/or enriched and/or extracted. For example, a coating can be provided at least on the inner wall that enhances the fixing of the at least one reagent to the inner wall of the container with the at least one additive, e.g. a material that can physically and/or chemically interact with the at least one additive. According to certain embodiments, the material of the container and/or optionally a coating is inert towards the mixture containing the at least one reagent and the at least one additive, and/or optionally towards a sample to be analyzed and/or enriched and/or extracted.

According to certain embodiments at least the inner wall of the container comprises a polymeric material. It is also possible that the further container comprises a polymeric material or that the whole container consists of a polymeric material, e.g. a thermoplastic material like polyethylene, polypropylene, polystyrene, polyvinylchloride, or mixtures or copolymers thereof. With the polymeric material an improved fixation of the at least one reagent to at least part of the inner wall of the container with the at least one additive is possible, e.g. by adhesion and/or van der Waals force. However, the container can also comprise glass or consist of glass.

The fixing of the at least one reagent to at least part of the inner wall of the container with the at least one additive is not particularly restricted as long as it can be avoided that the at least one reagent moves in the container and/or is dropped from the container when the container is moved, e.g. turned upside down, particularly when the container is centrifuged, e.g. upside down (with the at least one opening facing the ground) for 5 mins at a centrifugal force of 100g, e.g. with a Hettich Universal 320 R centrifuge when the container is a 2 mL Eppendorf tube that is put upside down in a 15 mL Falcon tube. According to certain embodiments, the mixture is fixed to at least part of the inner wall of the container in such a way that no components thereof are leaving the container, particularly a lump formed by the mixture, when the container is moved, e.g. turned upside down, particularly when the container is centrifuged, e.g. upside down (with the at least one opening facing the ground) for 5 mins at a centrifugal force of 100g, e.g. with a Hettich Universal 320 R centrifuge when the container is a 2 mL Eppendorf tube that is put upside down in a 15 mL Falcon tube.

By fixing the at least one reagent to the at least part of the inner wall of the container with the at least one additive the at least one reagent is not diluted by a solvent, which is especially of advantage for reagents which have to be used in a high concentration, e.g. 1 - 5 molar in water, e.g. 3 - 4 molar in water, in a method of extracting a substance from a sample, enriching a substance in a sample and/or detecting a substance in a sample, e.g. chaotropic salts in a method of extracting nucleic acids from a sample, e.g. blood sample.

The mixture containing the at least one reagent, and the at least one additive that is in solid form at room temperature is not particularly restricted as long as both the at least one reagent and the at least one additive are present in a combined form in the container. It is not excluded that they form separate phases, as long as both are in contact with each other and as long as the at least one reagent is fixed to at least part of the inner wall of the container with the at least one additive.

In the invention, the at least one reagent is dispersed in the at least one additive. This means that the at least one reagent forms a mixture with the at least one additive such that the at least one reagent is enclosed in, embedded in the at least one additive. The reagent therein preferably does not chemically react with the at least one additive when forming the mixture and/or when put into the container and/or during transport, storage, etc. Alternatively, it is also possible that the at least one reagent is returned to its original state when applying a sample to the mixture containing the at least one reagent, and the at least one additive. However, preferably the at least one additive does not change the at least one reagent when the mixture is formed and/or when the at least one reagent is dispersed in the at least one additive.

The formation of a dispersion is not particularly restricted as well as the state of dispersion of the at least one reagent in the at least one additive, and it is not excluded that some part of the reagent is in contact with the inner wall of the container and/or that a bigger volume of one or more reagents forms. It is also possible that the at least one reagent is dispersed in the at least one additive.

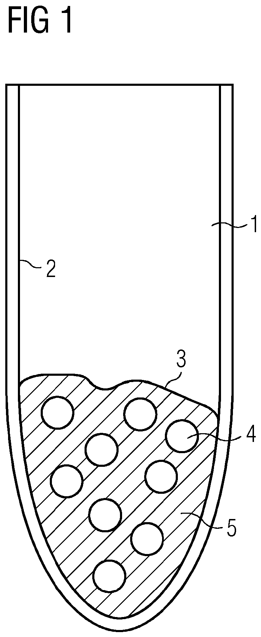

A schematic drawing of a container 1 having an inner wall 2 containing a mixture 3 of a reagent 4 and an additive 5 is shown in Fig. 1. In this figure the reagent 4 is covered in whole by the additive 5 and thus forms a dispersion of the reagent 4 in the additive 5. Fig. 1 thus shows a schematic view of a device according to the present invention. While the container 1 is shown in Fig. 1 in the form of a tube with a round bottom, it is not restricted to such a shape, though.

According to certain embodiments the at least one reagent and the at least one additive are included in the container in the form of a mixture that is essentially free of solvent. This does not exclude a case wherein at least one reagent is provided in liquid form, wherein e.g. liquid droplet are dispersed in the at least one additive. It is also not excluded that a solvent functions as at least one reagent, but such a case is not preferred, and the mixture of the at least one reagent and the at least one additive is preferably free of solvent. A solvent is this regard is a material that can dissolve another substance and can be an inorganic or organic, polar or non-polar solvent, e.g. water and/or an organic solvent like an alkane, an alcohol, an ester, an ether, a ketone, an amine, etc., and is not particularly restricted. A particular solvent is a polar solvent, e.g. water.

According to certain embodiments no solvents are included in the device. This means that the mixture as well as the container and any other optional parts of the device do not include a solvent. For example, the present device can be suitably packed or at least the at least one opening suitably closed, e.g. sealed, to avoid any ingress of a solvent, e.g. water.

According to certain embodiments the mixture comprises amorphous and/or crystalline phases. These can then be at least attributed to the at least one additive, which forms a solid, e.g. at a suitable temperature, e.g. room temperature, e.g. 20 - 25°C, e.g. 20, 21, 22, 23, 24 or 25°C, in which the at least one reagent or more than one reagents are fixed. In this instance the reagents themselves do not have to be solid and can be e.g. also liquid at that temperature, e.g. room temperature, e.g. 20 - 25°C, e.g. 20, 21, 22, 23, 24 or 25°C, e.g. in the case of detergents.

According to the present invention, the at least one additive is in solid form of sufficient dynamic viscosity µ of e.g. more than 1*10⁴ mPa·s, e.g. more than 1*10⁵ mPa·s, 1*10⁶ mPa·s, 1*10⁷ mPa·s, or 1*10⁸ mPa·s at room temperature when measured with a plate rheometer, e.g. is in solid form, at room temperature, e.g. 20 - 25°C, e.g. 20, 21, 22, 23, 24 and/or 25°C. According to certain embodiments, the device can be stored for a sufficient time at a temperature between -30 and 60 °C, e.g. between -20 and 50°C, e.g. between -10 and 45°C, e.g. between 0 and 40°C, e.g. at least of 20 - 25°C, e.g. 20, 21, 22, 23, 24 and/or 25°C for a sufficient time, e.g. at least one week, at least one month or at least one year, e.g. also upside-down with the at least one opening facing towards the ground without the mixture or a part thereof leaving the container, preferably without the mixture changing the shape thereof in the container.

According to certain embodiments the at least one additive comprises a water-soluble polymer, further preferably polyethylene glycol (PEG), polypropylene glycol (PPG), polyvinylpyrrolidone and/or polyvinyl alcohol, and/or copolymers thereof. According to certain embodiments, the at least one additive contains polyethylene glycol or is polyethylene glycol. According to certain embodiments, the molecular weight of the polymer is set in a range so that it is ductile at a temperature between -30 and 60 °C, e.g. between -20 and 50°C, e.g. be-tween -10 and 45°C, e.g. between 0 and 40°C, e.g. at least of 20 - 25°C, e.g. 20, 21, 22, 23, 24 and/or 25°C, e.g. to a weight average molecular weight between 500 and 10000, e.g. between 1000 and 8000, e.g. to about 6000, for polyethylene glycol. According to certain embodiments, the mixture containing the at least one reagent and the at least one additive, is ductile at a temperature between -30 and 60 °C, e.g. between - 20 and 50°C, e.g. be-tween -10 and 45°C, e.g. between 0 and 40°C, e.g. at least of 20 - 25°C, e.g. 20, 21, 22, 23, 24 and/or 25°C, to a degree that it does not separate from the at least part of the inner wall of the container during storage for a certain time, e.g. at least one week, at least one month or at least one year, e.g. also due to shrinkage.

With these additives, an improved binding of a substance to be enriched and/or extracted can e.g. be achieved, e.g. in the case of nucleic acids from a sample, e.g. by improving the bonding properties of nucleic acids to silica beads.

By selecting an additive with suitable properties (e.g. PEG with suitable molecular weight (weight average molecular weight) and therefore suitable melting point of e.g. 30 to 100°C, e.g. of 40 to 90°C, e.g. of 50 to 75°C, e.g. of about 60°C) the mixture of reagents and additive can be introduced, e.g. pressed, as a paste and/or at an increased temperature, e.g. 30 - 200°C, e.g. 35 - 180°C, e.g. 37 - 155°C, 38 to 150°C, e.g. 40 to 120°C, e.g. 50 to 95°C, into the container.

According to certain embodiments, the at least one additive, e.g. polyethylene glycol, has a solubility in water of at least 33 g/L at a temperature of 15°C to 25°C, e.g. of 20 - 25°C, e.g. 20, 21, 22, 23, 24 and/or 25°C, at normal pressure, i.e. 101325 Pa, preferably of at least 100 g/L, further preferably of at least 1000 g/L.

According to certain embodiments, the at least one additive can form a "wax-like" coating around the at least one reagent, e.g. particles, e.g. (crystal) particles of a chaotropic salt, and/or beads, e.g. magnetic silica-beads.

According to certain embodiments the at least one additive can form a coating around and/or a suspension with and/or a solution with a detergent and/or emulsifier, e.g. a liquid detergent and/or emulsifier like octoxinol 9 (Triton X-100) and/or polysorbates like Tween^{®} emulsifiers, and/or can absorb the detergent and/or emulsifier, e.g. a liquid detergent and/or emulsifier like octoxinol 9 (Triton X-100) and/or polysorbates like a Tween^{®} emulsifier, e.g. Tween^{®} 20, e.g. by solving thereof, preferably while keeping the "wax-like" properties.

According to certain embodiments, the at least one additive shows good adhesion to the inner wall of the container (e.g. polyethylene, polypropylene, polystyrene, polyvinylchloride, or mixtures or copolymers thereof). According to certain embodiments, the at least one additive is elastic and/or not brittle and/or shows no danger of cracking at a temperature between -30 and 60 °C, e.g. between -20 and 50°C, e.g. between -10 and 45°C, e.g. between 0 and 40°C, e.g. at least at room temperature, e.g. 20, 21, 22, 23, 24 and/or 25°C, and normal pressure.

In the present invention the at least one reagent is not particularly limited and can comprise one reagent or a mixture of two or more, e.g. three of more or four or more, reagents. If more than one reagent is comprised it is possible that different reagents can be contained in different layers of mixtures of additive and reagent, e.g. by introducing the different mixtures of at least one additive and at least one reagent one after the other into the container, e.g. layering them, so that different reagents can come into contact with the sample at different times upon introduction of the sample. In such an instance it is also possible that different additives are used in the different layers that are dissolved by a liquid of the sample at different times.

According to the present invention the at least one reagent comprises beads, particularly magnetic beads, e.g. magnetic silica beads, and/or at least one chaotropic agent like a chaotropic salt, urea, thiourea, etc., particularly a chaotropic salt like sodium dodecyl sulfate, lithium perchlorate, lithium acetate, magnesium chloride or a guanidinium salt, e.g. guanidinium chloride and/or guanidinium thiocyanate, and/or at least one detergent. According to certain embodiments, beads, e.g. magnetic silica beads, at least one chaotropic agent like a chaotropic salt, e.g. a guanidiunium salt, and at least one detergent, e.g. a polysorbate, are used as reagent. With this combination an extraction of nucleic acids from a sample, e.g. blood sample, is possible. According to certain embodiments, the at least one reagent contains beads, e.g. magnetic silica beads. According to certain embodiments, the at least one reagent contains a chaotropic agent, e.g. a chaotropic salt. According to certain embodiments, the at least one reagent contains a detergent and/or an emulsifier.

According to certain embodiments, the at least one reagent and/or the at least one additive do not have a notable vapor pressure, e.g. a vapor pressure below 2 kPa, e.g. below 1.8 kPa, e.g. below 1.7 kPa, e.g. below 1.6 kPa, e.g. below 1.5 kPa, e.g. below 1.4 kPa, e.g. also below 1 kPa, e.g. as measured by a Knudsen cell.

According to certain embodiments, the mixture of the at least one reagent and the at least one additive can form a compact phase with a solid outer surface. According to certain embodiments, this compact phase does not notably dissolve, e.g. with less than 10 g/L sample, e.g. less than 1 g/L sample, e.g. less than 0.1 g/L sample, for several minutes, e.g. 1, 2, 3, 4, 5, 6, 7, 8, 9, or 10 minutes, at room temperature, e.g. of 20 - 25°C, e.g. 20, 21, 22, 23, 24 and/or 25°C, upon addition of an aqueous sample without agitation of the mixture and/or sample. According to certain embodiments, the compact phase or at least the at least one additive dissolves upon addition of an aqueous sample and agitation and/or a temperature increase, e.g. to a temperature of 30 to 100°C, e.g. of 40 to 90°C, e.g. of 50 to 75°C, e.g. more than 60°C.

According to certain embodiments, the present device is a device for extracting nucleic acids from biological samples, consisting of:
- a container for receiving the sample, i.e. a sample material, and
- reagents that are fixed with an additive to an inner wall of the container.

Further, disclosed herein is a method of extracting a substance from a sample, enriching a substance in a sample and/or detecting a substance in a sample, comprising
- providing the device of the present invention, as disclosed above;
- introducing the sample into the container, e.g. filling the container with the sample, e.g. an aqueous sample like a blood sample or a sample derived therefrom; and
- mixing the sample with the mixture containing the at least one reagent, and the at least one additive. This method of extracting a substance from a sample is not part of the invention.

The mixing is carried out using mechanical agitation and/or a temperature increase, e.g. to a temperature of 30 to 100°C, e.g. of 40 to 90°C, e.g. of 50 to 75°C, e.g. of about 60°C. For introducing the sample to the container, an addition of a solvent like water to a sample is also possible prior to the introduction if the sample is a water-free or water-poor sample.

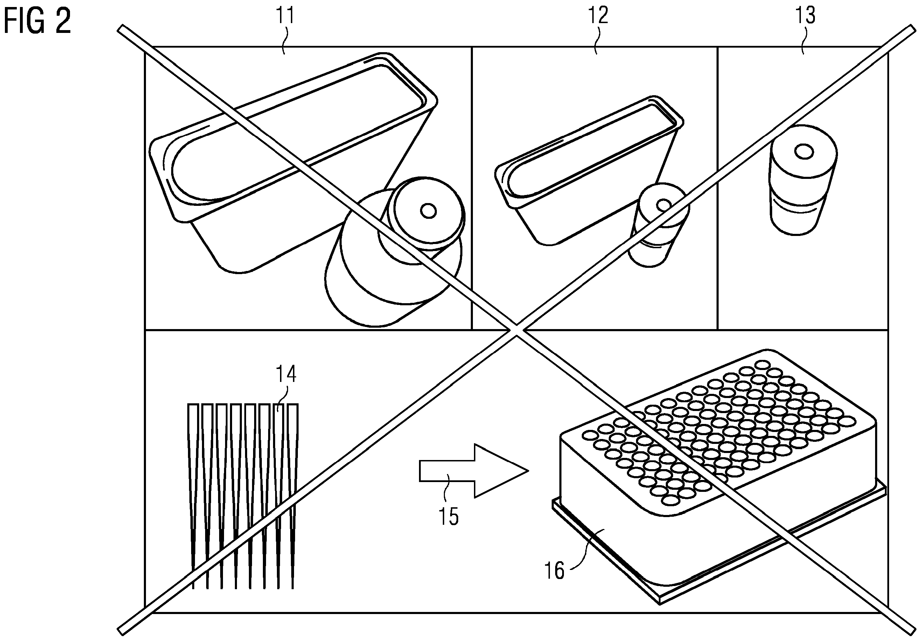

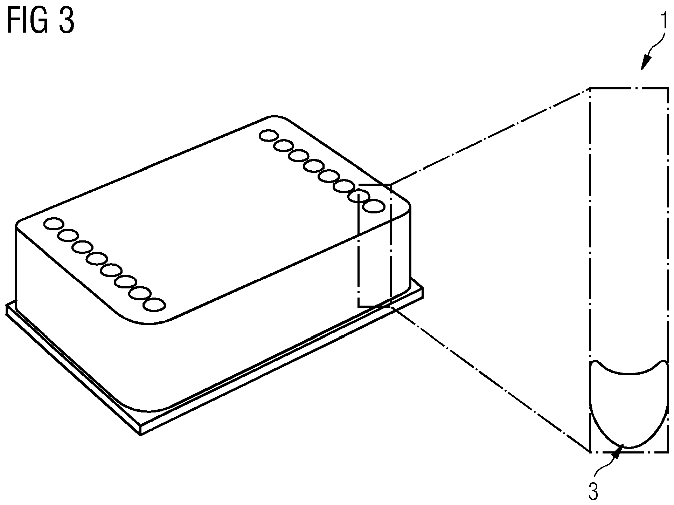

An exemplary embodiment of the method of extracting a substance from a sample, enriching a substance in a sample and/or detecting a substance in a sample, here extracting nucleic acids from a blood sample, is shown with regard to Fig. 3, whereas Fig. 2 shows requirements needed with regard to a method thereof of the state of the art.

In Fig. 2 and 3, the exemplary container is a 96-well titer plate, here a deep well plate 16, wherein the sample is introduced into the wells thereof, e.g. after introducing the mixture containing at least one reagent and at least one additive in the case of the present method.

A lysis buffer 11, beads 12 and an internal control 13 are exemplified as necessary agents in the embodiment shown in Fig. 2. These have to be decanted separately from screw-cap bottles to pipetting containers and then pipetted into the 96 wells using tips 14, e.g. 140 tips on average for a suitable extraction, taking about 30 minutes in pipetting step 15.

In contrast, in the method the mixture 3 containing the at least one reagent, e.g. the lysis buffer, beads and the internal control in case of the method shown in Fig. 2, and the at least one additive is introduced into the wells of the 96-well titer plate, wherein each well can be seen as a container 1, but also the whole plate can be seen as a container having 96 openings, as depicted in Fig. 3. Thus, the amount of liquid in the system can be minimized as well as the pipetting amount, with only one step of pipetting for introducing the sample in each respective well necessary. This way also the number of pipetting tips can be reduced.

Even filling of the 96-well plate with sample material takes several minutes e.g. with a 8-channel pipetting robot. This leads also to different starting times for the separate wells during extracting and/or enriching and/or analysis, which has to be accounted for in a quantitative method.

However, as the mixture of the at least one reagent and the at least one additive can form a compact phase with a solid outer surface in the present device, all 96 samples in the 96-well titer plate can be incubated and/or reacted at about the same time with the reagents, e.g. by starting a simultaneous agitation and/or temperature increase after introducing sample in all wells.

Furthermore, disclosed in another aspect of the present invention is a method of producing the present device, as discloses above, comprising:
- providing a container (1) having an inner wall (2) and at least one opening;
- preparing a mixture (3) containing at least one reagent comprising beads (4) and at least one additive (5) that is in solid form at room temperature; and
- introducing the mixture (3) in the container (1),

wherein the at least one reagent is fixed to at least part of the inner wall (2) of the container (1) with the at least one additive,
the at least one reagent is dispersed in the at least one additive (5), and
the at least one additive (5) is a water-soluble polymer selected from polyethylene glycol, polypropylene glycol, polyvinylpyrrolidone, polyvinylalcohol and/or copolymers thereof.

With this method the device of the present invention can be prepared. Thus, all features discussed with regard to the present device apply also to this method.

In this method, the preparation of the mixture containing at least one reagent and at least one additive is not particularly restricted, and the mixture can be prepared in any way possible, e.g. as a powder mixture, a suspension which can be "solidified" in the container, a mix, etc.

According to certain embodiments the mixture is introduced in the form of a melt and the melt is solidified. The forming of a melt is not particularly restricted, and a melt can be formed by e.g. heating the mixture containing the at least one reagent and the at least one additive to a suitable temperature, e.g. depending on the additive, wherein it is not excluded that some parts of the mixture are not molten. A suitable temperature can be e.g. 30 - 200°C, e.g. 50 - 180°C, e.g. 70 - 155°C, e.g. 75 - 130°C, e.g. 80 to 120°C.

According to certain embodiments the mixture is introduced in the form of a paste. Also the formation of the paste is not particularly restricted. For example, the at least one reagent can be introduced into a sufficiently ductile additive, e.g. by kneading, shaking, etc. For example, by selecting at least one additive with suitable properties (e.g. PEG with suitable molecular weight (weight average molecular weight) and therefore suitable melting point, as described above), the mixture of the at least one reagent and the at least one additive can be pressed as paste, e.g. at increased temperature of 30 to 80°C, e.g. of 40 to 75°C, e.g. of 50 to 70°C, e.g. of about 60°C, for example, into the container.

According to certain embodiments the mixture is introduced in the form of a powder mixture and subsequently heated, e.g. molten, in the container, e.g. by heating for a suitable time and at a suitable temperature, which can depend e.g. on the at least one additive. Subsequently the melt can be solidified, e.g. form a compact phase which is solid. A suitable temperature can be e.g. 30 - 200°C, e.g. 50 - 180°C, e.g. 70 - 155°C, e.g. 75 - 130°C, e.g. 80 to 120°C, and a suitable time between 30 seconds and 60 minutes, e.g. between 1 and 50 minutes, e.g. between 3 and 45 minutes, e.g. between 5 and 40 minutes, e.g. between 7 and 30 minutes.

According to certain embodiments the at least one reagent is introduced into the container and subsequently coated with the at least one additive. In such instance reagents can be introduced separately or as a mixture, e.g. a powder mixture, into the container if more than one reagent is introduced. The coating with the at least one additive is also not restricted, and the at least one additive can be introduced as powder and then melted in the container, e.g. as described above with regard to the introduction in the form of a powder mixture, or introduced as a melt, e.g. as described above with regard to the introduction in the form of a melt. It is not excluded that the mixture is then further heated until a compact phase is generated.

### Examples

The present invention will now be described in detail with reference to several examples thereof.

### Example 1

A magnetic silica-bead-suspension of Siemens VERSANT sample preparation 1.0 reagents kit (beads), Guanidine thiocyanate, BioUltra, SIGMA (GuSCN) (rubbed to powder in a porcelain mortar), and Tween^{®} 20, molecular biology grade, VWR (Tween) were added as reagents into a 2 ml Eppendorf tube (Eppi) together with poly(ethylene glycol) 6000, BioUltra, Fluka (PEG) (rubbed to powder in a porcelain mortar) as an additive, in the following manner.

25 µL beads are pipetted to the Eppendorf tube, magnetized and the solvent is pipetted off. 430 mg GuSCN are added, 65 mg PEG are added, and 50 mg Tween are added. The mixture is heated for 20 mins at 90°C. Then it is centrifuged hot at 12000 rpm in the tube in a miniSpin centrifuge for 1 min.

The surface of the mixture in the Eppendorf tube is slightly rough, GuSCN is coated by the PEG, and amorphous and crystalline phases can be observed.

After cooling the tube with the mixture, it is centrifuged "upside down" with a Hettich Universal 320 R centrifuge (the 2 mL Eppendorf tube with the mixture is put upside down in 1 15 mL Falcon tube) for 5 mins at a centrifugal force of 100g. With such centrifugation no change is observed for the mixture in the Eppendorf tube. After 5 mins in the same centrifuge at 200g the mixture is detached from the tube.

This shows that the mixture can be suitably fixed to the Eppendorf tube for storage.

### Example 2

A tube as prepared in Example 1 was used for testing dissolution of the mixture in a sample. As sample, 500 µL blood plasma were pipetted on the surface of the mixture in the Eppendorf tube. The sample was then left for 5 minutes, during which time no visible change was observed for the mixture.

Thereafter, the tube with the sample liquid was put in a heater-shaker at a temperature of 60°C and at 1000 rpm. Within 5 minutes the mixture completely dissolved in the blood plasma.

Thereafter, the nucleic acids in the blood plasma were enriched in a usual manner with the reagents contained in the mixture.

## Claims

1. Device, comprising a container (1) having an inner wall (2) and at least one opening, containing a mixture (3) containing
- at least one reagent comprising beads (4), and
- at least one additive (5) that is in solid form at room temperature,
wherein the at least one reagent is fixed to at least part of the inner wall (2) of the container (1) with the at least one additive (5),
the at least one reagent is dispersed in the at least one additive (5), and
the at least one additive (5) is a water-soluble polymer selected from polyethylene glycol, polypropylene glycol, polyvinylpyrrolidone, polyvinylalcohol and/or copolymers thereof.

2. Device according to claim 1, wherein the at least one reagent and the at least one additive (5) are included in the container (1) in the form of a mixture (3) that is essentially free of solvent.

3. Device according to claim 1 or 2, wherein no solvents are included in the device.

4. Device according to any one of the preceding claims, wherein the mixture (3) comprises amorphous and/or crystalline phases.

5. Device according to any one of the preceding claims, wherein the container (1) is a tube, particularly a microtube, a well plate, or a bottle.

6. Device according to any one of the preceding claims, wherein the beads (4) comprise magnetic beads, e.g. magnetic silica beads.

7. Device according to any one of the preceding claims, wherein the at least one reagent comprises at least one chaotropic agent, particularly a chaotropic salt, and/or at least one detergent.

8. Device according to any one of the preceding claims, wherein at least the inner wall (2) of the container (1) comprises a polymeric material.

9. Use of the device according to any one of claims 1 to 8 for enriching nucleic acid in a blood sample and/or detecting a nucleic acid in a blood sample.

10. Method of producing the device according to any one of claims 1 to 8, comprising:
- providing a container (1) having an inner wall (2) and at least one opening;
- preparing a mixture (3) containing at least one reagent comprising beads (4) and at least one additive (5) that is in solid form at room temperature; and
- introducing the mixture (3) in the container (1),
wherein the at least one reagent is fixed to at least part of the inner wall (2) of the container (1) with the at least one additive,
the at least one reagent is dispersed in the at least one additive (5), and
the at least one additive (5) is a water-soluble polymer selected from polyethylene glycol, polypropylene glycol, polyvinylpyrrolidone, polyvinylalcohol and/or copolymers thereof.

## Patentansprüche

1. Vorrichtung, umfassend einen Behälter (1) mit einer Innenwand (2) und mindestens einer Öffnung, enthaltend eine Mischung (3), enthaltend:
- mindestens ein Reagenz, umfassend Perlen (4), und
- mindestens ein Additiv (5), das bei Raumtemperatur in fester Form vorliegt,
wobei das mindestens eine Reagenz an mindestens einem Teil der Innenwand (2) des Behälters (1) mit dem mindestens einen Additiv (5) fixiert ist,
das mindestens eine Reagenz in dem mindestens einen Additiv (5) dispergiert ist, und
das mindestens eine Additiv (5) ein wasserlösliches Polymer ausgewählt aus Polyethylenglykol, Polypropylenglykol, Polyvinylpyrrolidon, Polyvinylalkohol und/oder Copolymeren davon ist.

2. Vorrichtung nach Anspruch 1, wobei das mindestens eine Reagenz und das mindestens eine Additiv (5) in den Behälter (1) in Form einer Mischung {3} eingeschlossen sind, die im Wesentlichen frei von Lösungsmittel ist.

3. Vorrichtung nach Anspruch 1 oder 2, wobei in die Vorrichtung keine Lösungsmittel eingeschlossen sind.

4. Vorrichtung nach einem der vorhergehenden Ansprüche, wobei die Mischung (3) amorphe und/oder kristalline Phasen umfasst.

5. Vorrichtung nach einem der vorhergehenden Ansprüche, wobei der Behälter (1) ein Röhrchen, insbesondere ein Mikroröhrchen, eine Well-Platte oder eine Flasche ist.

6. Vorrichtung nach einem der vorhergehenden Ansprüche, wobei die Perlen (4) magnetische Perlen umfassen, z. B. magnetische Silikaperlen.

7. Vorrichtung nach einem der vorhergehenden Ansprüche, wobei das mindestens eine Reagenz mindestens ein chaotropes Mittel, insbesondere ein chaotropes Salz, und/oder mindestens ein Detergens umfasst.

8. Vorrichtung nach einem der vorhergehenden Ansprüche, wobei mindestens die Innenwand (2) des Behälters (1) ein polymeres Material umfasst.

9. Verwendung der Vorrichtung nach einem der Ansprüche 1 bis 8 zum Anreichern von Nukleinsäure in einer Blutprobe und/oder Detektieren einer Nukleinsäure in einer Blutprobe.

10. Verfahren zur Produktion der Vorrichtung nach einem der Ansprüche 1 bis 8, umfassend:
- Bereitstellen eines Behälters (1) mit einer Innenwand (2) und mindestens einer Öffnung;
- Herstellen einer Mischung (3), die mindestens ein Reagenz, umfassend Perlen (4), und mindestens ein Additiv (5) enthält, das bei Raumtemperatur in fester Form vorliegt; und
- Einbringen der Mischung (3) in den Behälter (1), wobei das mindestens eine Reagenz an mindestens einem Teil der Innenwand (2) des Behälters (1) mit dem mindestens einen Additiv fixiert ist,
das mindestens eine Reagenz in dem mindestens einen Additiv (5) dispergiert ist, und
das mindestens eine Additiv (5) ein wasserlösliches Polymer ausgewählt aus Polyethylenglykol, Polypropylenglykol, Polyvinylpyrrolidon, Polyvinylalkohol und/oder Copolymeren davon ist.

## Revendications

1. Dispositif, comprenant un récipient (1) ayant une paroi interne (2) et au moins une ouverture, contenant un mélange (3) contenant
- au moins un réactif comprenant des billes (4), et
- au moins un additif (5) qui est sous forme solide à température ambiante,
dans lequel ledit au moins un réactif est fixé sur au moins une partie de la paroi interne (2) du récipient (1) avec ledit au moins un additif (5),
ledit au moins un réactif est dispersé dans ledit au moins un additif (5), et
ledit au moins un additif (5) est un polymère hydrosoluble choisi parmi le polyéthylène glycol, le polypropylène glycol, la polyvinylpyrrolidone, l'alcool polyvinylique et/ou leurs copolymères.

2. Dispositif selon la revendication 1, dans lequel ledit au moins un réactif et ledit au moins un additif (5) sont inclus dans le récipient (1) sous la forme d'un mélange (3) qui est essentiellement dépourvu de solvant.

3. Dispositif selon la revendication 1 ou 2, dans lequel aucun solvant n'est inclus dans le dispositif.

4. Dispositif selon l'une quelconque des revendications précédentes, dans lequel le mélange (3) comprend des phases amorphes et/ou cristallines.

5. Dispositif selon l'une quelconque des revendications précédentes, dans lequel le récipient (1) est un tube, notamment un microtube, une plaque à puits, ou un flacon.

6. Dispositif selon l'une quelconque des revendications précédentes, dans lequel les billes (4) comprennent des billes magnétiques, par exemple des billes de silice magnétique.

7. Dispositif selon l'une quelconque des revendications précédentes, dans lequel ledit au moins un réactif comprend au moins un agent chaotropique, notamment un sel chaotropique, et/ou au moins un détergent.

8. Dispositif selon l'une quelconque des revendications précédentes, dans lequel au moins la paroi interne (2) du récipient (1) comprend un matériau polymère.

9. Utilisation du dispositif selon l'une quelconque des revendications 1 à 8 pour enrichir un acide nucléique dans un échantillon de sang et/ou détecter un acide nucléique dans un échantillon de sang.

10. Procédé de fabrication du dispositif selon l'une quelconque des revendications 1 à 8, comprenant les étapes consistant à :
- fournir un récipient (1) comportant une paroi interne (2) et au moins une ouverture ;
- préparer un mélange (3) contenant au moins un réactif comprenant des billes (4) et au moins un additif (5) qui est sous forme solide à température ambiante ; et
- introduire le mélange (3) dans le récipient (1),
dans lequel ledit au moins un réactif est fixé à au moins une partie de la paroi interne (2) du récipient (1) avec ledit au moins un additif,
ledit au moins un réactif est dispersé dans ledit au moins un additif (5), et
ledit au moins un additif (5) est un polymère hydrosoluble choisi parmi le polyéthylène glycol, le polypropylène glycol, la polyvinylpyrrolidone, l'alcool polyvinylique et/ou leurs copolymères.
